(19) Europäisches Patentamt
European Patent Office
Office européen des brevets

(11) Publication number: 0 252 766 B1

(12) EUROPEAN PATENT SPECIFICATION

(45) Date of publication of the patent specification: 10.01.90

(51) Int. Cl.[4]: C07C 57/52, C07C 57/54, C07C 51/15

(21) Application number: 87306178.2

(22) Date of filing: 13.07.87

(54) A method for preparing fluorine containing alpha,beta-unsaturated carboxylic acids.

(30) Priority: 11.07.86 JP 163359/86

(43) Date of publication of application: 13.01.88 Bulletin 88/2

(45) Publication of the grant of the patent: 10.01.90 Bulletin 90/2

(84) Designated Contracting States: CH DE FR GB IT LI

(56) References cited:
GB-A- 756 385
US-A- 3 311 657
US-A- 4 098 806

(73) Proprietor: TOSOH CORPORATION, 4560, Oaza-Tonda Shinnanyo-shi, Yamaguchi 746(JP)

(72) Inventor: Takahashi, Mitsuru, 3056-1, Oaza Tonda, Shinnanyo-shi Yamaguchi-ken(JP)
Inventor: Shuyama, Hideo, 4073-10, Oaza Mure, Hofu-shi Yamaguchi-ken(JP)
Inventor: Oyama, Kiyotaka, 7-15-12, Nijigaoka, Hiraki-shi Yamaguchi-ken(JP)

(74) Representative: Sheard, Andrew Gregory et al, Kilburn & Strode 30, John Street, London WC1N 2DD(GB)

EP 0 252 766 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to method for the preparation of fluorine-containing alpha, beta-unsaturated carboxylic acids containing fluorine; more particularly the invention relates to a rapid and efficient method for producing fluorine-containing alpha, beta-unsaturated carboxylic acids starting from fluorine-containing alkenyl halides as the raw material.

Alpha, beta-unsaturated carboxylic acids containing fluorine are useful substances as intermediate raw materials for various kinds of fluorine-containing, for example, synthetic intermediates for pharmacological and agricultural medicaments, paint materials and raw materials for polymers used in resists for the production of LSIs, etc.

Conventional methods for preparing fluorine-containing alpha, beta-unsaturated carboxylic acids starting from alkenyl halides are as follows:

1) starting from fluorine-containing vinyl bromide and n-butyl lithium or magnesium, fluorine or magnesium bromide-containing vinyl lithium is prepared and reacted with carbon dioxide at low temperatures (J. Org. Chem.) 33 280 (1967) or (Chem. Abs.) 53 6987 g);
2) fluorine-containing vinyl halide compounds are reacted with carbon dioxide in the presence of a Zn - Cu pair (Japanese Patent Publication No. Sho 60-6332), or are irradiated with ultrasonic waves in the presence of zinc powder (10th Fluorine Chemistry Pannel Discussion, 1985, p33);
3) in the presence of triethylamine and palladium catalyst, 2-bromo-3,3,3-trifluoropropene, carbon monoxide, and water are reacted to synthesize alpha-trifluoromethyl acrylic acid (Japanese Laid-Open Patent Applications No. Sho 58-154529, No. Sho 60-94933).

It is hard to say, however, that these conventional methods are industrially adequate. In the method using n-butyl lithium or magnesium, reaction must be carried out at low tempereatures such as -100°C or -40°C and the yield of product is low.

In the method using a Zn - Cu pair there are some problems: the Zn - Cu must be prepared prior to a reaction, so that the reaction procedure will be complicated, and it is difficult to prepare a Zn - Cu pair possessing a given activity, so reproducibility of a yield can be obtained. Further, in the method wherein reaction is carried out in the presence of zinc powder with irradiating ultrasonic waves, it is difficult to obtain a large enough ultrasonic wave generating apparatus, and the yield is low. Therefore, from the industrial point of view it is hard to say that this method is efficient. In the method using a palladium catalyst, the catalyst is expensive and carbon monoxide has some safety problems; hence it is hard to adopt this method as a synthetic industrial one.

In short, the conventional methods relating to the preparation of alpha, beta-unsaturated carboxylic acids containing fluorine starting from alkenyl halides containing fluorine have had the following problems:

1) reaction conditions are severe,
2) yield is low,
3) the reaction procedure is complicated.

From the aforesaid circumstances, intensive investigations were made by the present inventors to provide a high yield method for preparing fluorine-containing alpha, beta-unsaturated carboxylic acids under simple and mild conditions.

Finally, in a method for reacting an alkenyl halide containing fluorine with carbon dioxide in the presence of zinc, the present inventors found that cations coexisting in the reaction system greatly improve the yield, and they thus made the present invention. A feature of the present invention is to react an alkenyl halide containing fluorine represented by general formula (I): $R_1(R_2)C=C(R_3)X$ wherein $R_1$, $R_2$, and $R_3$ are hydrogen, fluorine, alkyl, or alkyl containing fluorine (i.e fluoroalkyl), X is chlorine, bromine, or iodine) with carbon dioxide in an organic solvent and in the presence of zinc and at least one cation selected from alkali metal ions, alkaline earth metal ions, and ammonium ions in the reaction system, and then to hydrolyze the reaction product to obtain a fluorine-containing alpha, beta-unsaturated carboxylic acid represented in general formula (II):$R_1(R_2)C=C(R_3)CO_2$

Various fluorine-containing alkenyl halides as represented in general formula (I) can be used in a method of the present invention and any one of $R_1$, $R_2$, and $R_3$ represented in general formula (I) should be fluorine or a fluorine-containing alkyl group on the basis of reactivity of alkenyl halide and zinc. Examples of such compounds are fluorine-containing vinyl halide compounds as represented in $F_2C=C(F)X$, $F_2C=C(H)X$, $H(F)C=C(F)X$, $H(F)C=C(H)X$, and $H_2C=C(F)X$ (wherein X is any of chlorine, bromine, or iodine), or fluorine containing 1-or 2-alkenyl halides containing fluorine, wherein hydrogen or fluorine of those compounds is substituted for alkyl or fluorine-containing alkyl, as represented in $F_2C=C(R)X$, $F(R)C=C(F)X$, $H(R)C=C(F)X$, $F(R)C=C(H)X$, $H(F)C=C(R)X$, $F_2C=C(Rf)X$, $F(Rf)C=C(F)X$, $H(Rf)C=C(F)X$ $F(Rf)C=C(H)X$, $H(F)C=C(Rf)X$, $H_2C=C(Rf)X$, $R_2C=C(F)X$, $Rf_2C=C(F)X$, $Rf(F)C=C(F)X$, $Rf_2C=C(H)X$, $Rf(R)C=C(H)X$ (wherein R is an alkyl group; Rf is fluorine containing alkyl; X is chlorine, bromine, or iodine), and various substitution products of R and Rf as represented in

F(R)C=C(R)X, Rf(F)C=C(Rf)X, $Rf_2C$=C(Rf)X, Rf(R)C=C(R)X, $R_2C$=C(Rf)X, $Rf_2C$=C(R)X, Rf(R)C=C(Rf)X, R(F)C=C(Rf)X, Rf(F)C=C(R)X, RF(H)C=C(Rf)X, Rf(H)C=C(R)X, R(H)C=C(Rf)X (wherein R is alkyl, Rf is fluorine-containing alkyl, and X is chlorine, bromine, or iodine). In this case, taking into considerations on the solvent solubility of fluorine containing alkenyl halides, the number of carbon atoms in the alkyl group or fluorine containing alkyl is for example less than 30 and preferably not more than 20. Further, any fluorine containing alkyl group containing fluorine possessing the effect of a substituent group similar to the trifluoromethyl group can be used, but a perfluoro or polyfluoro aliphatic group having a straight or branched chain is preferable.

In general, alkyl groups preferably have 1 to 30, e.g. 1 to 20, for example 1 to 10 or 1 to 6 carbon atoms and alkenyl groups preferably have 2 to 30, e.g. 2 to 20, for example, 2 to 10 or 2 to 6 carbon atoms.

In the method of present invention, at least one sort of cation selected from alkali metal ions, alkaline earth metal ions and ammonium ions should coexist in a reaction.

In the present invention, an ammonium ion means $NH_4^+$ or one that all or some of the hydrogen atoms in $NH_{4+}$ are substituted by at least one sort of substituent selected from (e.g. $C_{1-6}$) alkyl, (e.g. $C_6$ or $C_{10}$) aryl, or (e.g. $C_{2-6}$ or $C_{3-6}$) alkylene, or a cation wherein a substituent selected from hydrogen, (e.g. $C_{1-6}$) alkyl, or (e.g. $C_6$ or $C_{10}$) aryl combines to a nitrogen atom of pyridine.

Examples of such cations are as follows:

$Li^+$, $Na^+$, $K^+$, $Rb^+$, $Cs^+$, $Be^{2+}$, $Mg^{2+}$, $Ca^{2+}$, $Sr^{2+}$, $Ba^{2+}$, $NH_4^+$, $N(CH_3)_4^+$, $N(C_2H_5)_4^+$, $N(n\text{-}C_3H_7)_4^+$, $N(n\text{-}C_4H_9)_4^+$, $NH(CH_3)_3^+$ $NH(C_2H_5)_3^+$, $NH(n\text{-}C_3H_7)3^+$, $NH(n\text{-}C_4H_9)_3^+$, $\theta\text{-}NH_3+$, (pyridyl)-$H^+$.

These cations can be applied to the present invention individually in combination of several sorts. Furthermore, these cations can be easily supplied by addition of compounds consisting of these cations and anions to the reaction system.

Examples of anions, not restricted especially, are as follows:

$F^-$, $Cl^-$, $Br^-$, $I^-$, $OH^-$, $O^{2-}$, $Cl0_2^-$, $Cl0_3^-$, $Cl0_4^-$, $BF_4^-$, $S_2O_3^{2-}$, $SO4^{2-}$, $NO_2^-$, $NO_3^-$, $N_3^-$, $PO_4^{2-}$, $CH_3O^-$, $C_2H_5O^-$, $CH_3S^-$, $CN^-$, $SCN^-$, $CO_3^{2-}$, $HCO_3^-$, $CH_3COO^-$, $C_2O_4^{2-}$.

The amount of cations to coexist is preferably within the range from 0.01 to 50 gram atoms against 1 mol general formula (I). An amount of cation less than 0.01 gram atom has a reduced yield promoting effect and an amount of more than 50 gram atoms results has no effect of promoting yield in proportion to the amount of cation coexisting.

Zinc used in the method of the present invention may be employed in the form of powder, the mean size of particles in diameter preferably ranging from 0.1 to 100 micrometres (mcm) in diameter. If the particle size is less than 0.1 mcm in diameter, the procedure for removing it after the reaction will be complicated, and if it is more than 100 mcm in diameter, the reaction yield will be reduced due to the decrease in the effective area available during the reaction. Taking yield and procedure into consideration, the mean size of particles is preferably, in particular, 1 to 50 mcm in diameter.

The amount of zinc can be decreased by pretreating the surface of the zinc, though commercially available zinc powder can be utilized without treatment. The surface treatment method may be carried out according to the method by Houben-Weyl (Houben-Weyl), 13 (2a), 570 -574, 815): pretreatment with acid treatment agents (mineral acids or acetic acid) and formation of metal pairs with other metals, e.g., in general, copper, lead, cadmium, mercury, etc.

Zinc powder may be used in the amount from 1 to 10 equivalents compared to the alkenyl halide containing fluorine, preferably 2 to 10 equivalents, to get good reproducibility.

Preferred solvents in methods of the present invention are aprotic polar solvents, e.g. DMF, DMSO, N,N-dimethylacetamido, tetramethyl urea, hexamethylphosphoramide, sulphorane, N-methylpyrrolidone, nitrobenzene, nitromethane, acetonitrile, propylene carbonate, tetrahydrofuran, dioxan, ether, diglyme, triglyme, pyridine. From a yield point of view, the following are desirable: DMF, DMSO, N-methylpyrrolidone, hexamethylphosphoramide.

The reaction in the present invention can be carried out within a wide range of temperature, preferably 0 to 150°C. Since temperatures below 0°C lead to a long reaction time to promote conversion of the raw material, i.e. the alkenyl halide containing fluorine, it is not practical. Temperatures above 150°C lower the objective selectivity coefficiency to carboxylic acid considerably, because of the increase in the proportions of side-reactions.

The reaction can be carried out by contacting the aforesaid fluorine containing alkenyl halide with carbon dioxide in the presence of cations in an organic solvent, and in a suspension of zinc at a given temperature. To supply carbon dioxide into the reaction system, various ways can be adopted.

There are, for example, a technique where carbon dioxide is introduced through a leading tube into the system and a technique where carbon dioxide is dissolved in a solvent compulsively under pressure.

Taking yield into consideration, the alkenyl halide containing fluorine should preferably be added into the system at a given temperature in the presence of zinc, a solvent, carbon dioxide, and cations. It may be added all at once, but alternatively the raw material can be introduced into the system at a constant

rate. Practically, the rate is desirably within the range of from 0.01 to 10 mol/hr per 1 litre of solvent.

The reaction time is suitably for 30 min to 100 hr after finishing the addition of the fluorine containing alkenyl halide. However, in the case that the fluorine containing alkenyl halide is a solid and the aforesaid way cannot be adopted due to the alkenyl halide's low solvent solubility, the reaction can be carried out in such a way that solvent is added to the system where in zinc and halides have been mixed with the cation containing compound in advance under an atmosphere of carbon dioxide. In this way, the reaction time is suitably 30 min to 100 hr after finishing the addition of solvent and setting the system a given temperature.

As mentioned above, the object compounds, fluorine-containing alpha,beta-unsaturated carboxylic acids can be obtained by hydrolysis of the reaction product after reaction of the fluorine containing alkenyl halide containing fluorine with carbon dioxide in the presence of zinc and cations. Hydrolysis proceeds simply by contacting the reaction mixture with a mineral acid such as hydrochloric acid, sulphuric acid or nitric acid.

In methods of the present invention, the production of side-products is extremely low, so that fluorine containing alkenyl halides containing fluorine can be changed in high yield into the object alpha, beta-unsaturated carboxylic acids.

Furthermore, procedures for recovery of unreacted raw material become unnecessary and product purification processes are simplified which means that the isolation procedure is simplified.

The following Examples and Comparison Examples will illustrate the present invention in detail, but the present invention is not limited to them.

Example 1

Into a 200 cc magnetic stirrer type autoclave, which is provided withthe inlet port for carbon dioxide and inlet port for 3,3,3-trifluoro-2-bromopropene, 7.85 g (0.12 gram atom) of zinc powder (mean particle diameter about 15 mcm) which is washed with 0.2 N-HC1 solution and dried in advance and 2.55 g (60 mmol) of lithium chloride were introduced and the temperature inside the autoclave was raised to 35°C by heating. The pressure of carbon dioxide was kept at 6.0 Kg/$cm_2$ (absolute pressure) by means of a constant pressure apparatus until completion of the reaction.

Eighty ml of DMF was then introduced into the autoclave, with stirring, by the use a transfer pump. DMF poured into the autoclave begins to dissolve carbon dioxide and the concentration finally shows a saturated dissolution concentration (1 mol/1) under a gaseous carbon dioxide pressure of 6.0 Kg/$cm_2$ (absolute pressure).

The mixture of 7.0 g (40 mmol) of 3,3,3-trifluoro-2-bromopropene and 26 ml of DMF was then introduced into the autoclave by the use of a transfer pump over about ten min. After that, 10ml of DMF was further introduced to wash the inside of the tube. After stirring for 24 hr at the same temperature, the pressure inside the autoclave was returned to atmospheric pressure and the reaction was complete.

A gas-chromatograph analysis of the reaction solution showed 100 percent conversion: the 3,3,3-trifluoro-2-bromopropene raw material was all ccnsumed.

After removing solids from the reaction mixture by filtration, the filtrate was poured into 250 ml of 6N-HC1 solution and the intermediate product was hydrolysed. A diethyl ether extract was then analyzed by gas-chromatography. The results showed that the object alpha-trifluoromethyl acrylic acid was prepared in the yield of 79%.

Examples 2 - 9

The same procedure as Example 1 was followed, except that the alkali metal ion containing compound shown in Table 1 was used instead of lithium chloride as a cation source in a molar ratio of 1.5 as compared to the 3,3,3-trifluoro-2-bromopropene.

The results are summarised in Table 1.

Table 1

| Example | Compound added | Conversion (%) | Yield (%) |
|---|---|---|---|
| 2 | NaI | 100 | 72 |
| 3 | KI | 87 | 75 |
| 4 | NaBr | 100 | 72 |
| 5 | KBr | 100 | 74 |
| 6 | NaCl | 100 | 71 |
| 7 | KCl | 100 | 72 |
| 8 | KF | 100 | 74 |
| 9 | $CH_3COONa$ | 100 | 76 |

Comparative Example 1

The same procedure as Example 1 was followed except that lithium chloride was not used. The yield of alpha-trifluoromethyl acrylic acid prepared was 57%.

Examples 10 - 12

The same procedure as Example 1 was followed except that the alkaline earth metal ion containing compound shown in Table 2 was used instead of lithium chloride as a cation source in a molar ratio of as compared to the 3,3,3-trifluoro-2-bromopropene.

The result are summarized in Table 2.

Table 2

| Example | Compound added | Conversion (%) | Yield (%) |
|---|---|---|---|
| 10 | $MgCl_2$ | 100 | 72 |
| 10 | $BACl_2$ | 100 | 73 |
| 12 | $MgBr_2$ | 100 | 71 |

Example 13

The same procedure as Example 1 was followed except that the reaction temperature was 15°C instead of 35°C and the carbon dioxide pressure was 11 $Kg/cm_2$ instead of 6 $Kg/cm_2$.

The yield of alpha-trifluoromethyl acrylic acid prepared was 92%.

Example 14

The same procedure as Example 1 was followed except that the carbon dioxide was atmospheric.

The yield of alpha-trifluoromethy acrylic acid was 70%.

Examples 15, 16

The same procedure as Example 1 was followed except that the ammonium ion containing compound shown in Table 3 was used in place of the lithium chloride as a cation source in a molar ration of 1.5 as compared to the 3,3,3-trifluoro-2-bromopropene.

The resuls are summarized in Table 3.

Table 3

| Example | Compound added | Conversion (%) | Yield (%) |
|---|---|---|---|
| 15 | $N(CH_3)_4Br$ | 100 | 70 |
| 16 | $N(C_2H_5)_4I$ | 100 | 73 |

Example 17

The same procedure as Example 1 was followed except that the reaction time was 5 hr instead of 24 hr.

The 3,3,3-trifluoro-2-bromopropene raw material was all consumed after the reaction, so the conversion was shown to be 100%.

The yield of alpha-trifluoromethyl acrylic acid was 76%.

Example 18

The same procedure as Example 1 was carried out, except that 8.9 g (40 mmol) of 3,3,3-trifluoro-2-iodopropene was used instead of 7.0 g (40 mmol) of 3,3,3-trifluoro-2-bromoproene.

The yield of alpha-trifluoromethyl acrylic acid obtained was 89%.

Examples 19 - 22

The same procedure as Example 1 was carried out, except that the 3,3,3-trifluoro-2-bromopropene was replaced by 40 mmol of 3,3,4,4,5,5,6,6,6-nonafluoro-2-bromohexene, 1,2,2-trifluoro-1-iodoethene, 1,2-difluoro-1-iodo-3-methylpentene, or 2,3,3,4,4,5,5,6,6,7,7,8,8,9,9,9-hexadecafluoro-1-iodononene. The corresponding alpha, beta-unsaturated carboxylic acids were prepared. The products were identified by IR, NMR, and so on.

The results are indicated in Table 4.

Table 4

| Example | Product | Yield (%) |
|---|---|---|
| 19 | $n\text{-}C_4F_9C(CO_2H){=}CH_2$ | 63 |
| 20 | $CF_2{=}CF.CO_2H$ | 91 |
| 21 | $CH_3CH_2CH(CH_3)CF{=}CH.CO_2H$ | 87 |
| 22 | $n\text{-}C_7F_{15}CF{=}CHCO_2H$ | 85 |

Comparative Examples 2 - 5

The same procedure as Examples 19 - 22 was carried out except that lithium chloride was not added.

The results are summarized in Table 5.

Table 5

| Comparison Example | Product | Yield (%) |
|---|---|---|
| 2 | $n\text{-}C_4F_9C(CO_2H){=}CH_2$ | 42 |
| 3 | $CF_2{=}CF.CO_2H$ | 79 |
| 4 | $CH_3CH_2CH(CH_3)CF{=}CF.CO_2H$ | 72 |
| 5 | $n\text{-}C_7F_{15}CF{=}CHCO_2H$ | 72 |

## Claims

1. A method for the preparation of an alpha, beta-unsaturated carboxylic acid of the general formula

$R^1(R^2)C{=}C(R^3)CO_2H$

wherein each of $R^1$, $R^2$, and $R^3$ independently represents Hydrogen, fluorine, alkyl, or fluoroalkyl and at least one of $R^1$, $R^2$, and $R^3$ is fluorine or fluoroalkyl, the process comprising:
(a) reacting fluorine containing alkenyl halide of the general formula

$R^1(R^2)C{=}C(R^3)X$

wherein $R^1$, $R^2$, and $R^3$ are same as above and X is chlorine, bromine, or iodine, with carbon dioxide in an organic solvent and in the presence of zinc and at least one cation selected from alkaline earth metal ions, alkali metal ions, and ammonium ions, and
(b) hydrolyzing the resulting product

2. A method according to Claim 1, wherein the fluoroalkyl group is a perfluoro or polyfluoro aliphatic group with a $C_{1-20}$ straight or branched chain.

3. A method according to Claim 1 or 2, wherein from 1 to 10 equivalents of zinc are used compared to the fluorine containing alkenyl halide.

4. A method according to Claim 1, 2 or 3, wherein the reaction is carried out at a temperature ranging from 0 to 150°C.

5. A method according to any of one of Claims 1 to 4, wherein the said cation is present in an amount of from 0.01 - 50 gram atoms per 1 mol of the fluorine containing alkenyl halide.

6. A method according to any one of Claims 1 to 5, wherein the zinc is activated zinc.

7. A method according to any one of Claims 1 to 6, wherein the alkyl or fluoroalkyl group of the fluorine-containing alkenyl halide contains 20 or less carbon atoms.

8. A method according to any one of Claims 1 to 7, wherein the fluorine containing alkenyl halide is 3,3,3-trifluoro-2-bromopropene, 3,3,4,4,5,5,6,6,6-nonafluoro-2-bromohexene, 1,2,2-trifluoro-1-iodoethene, 1,2-difluoro-1-iodo-3-methylpentene, or 2,3,3,4,4,5,5,6,6,7,7,8,8,9,9,9-hexadecafluoro-1-iodononene.

9. A method according to any one of Claims 1 to 8, organic solvent is selected from DMF, DMSO, N-methylpyrrolidone, N,N-dimethylacetamide, tetramethyl urea, and hexamethylphosphoramide.

10. A method for the preparation of an alpha, beta-unsaturated carboxylate salt of the general formula

$R^1(R^2)C{=}C(R^3)CO_2M$

wherein each $R^1$, $R^2$ and $R^3$ independently represents Hydrogene, fluorine, alkyl, or fluoroalkyl and at least one of $R^1$, $R^2$, and $R^3$ is fluorine or fluoroalkyl, and M represents a cation, the process comprising reacting fluorine containing alkenyl halide of the general formula

$R^1(R^2)C{=}C(R^3)X$

wherein $R^1$, $R^2$, and $R^3$ are same as above and X is chlorine, bromine, or iodine, with carbon dioxide in an organic solvent and in the presence of zinc and at least one cation selected from alkaline earth metal ions, alkali metal ions, and ammonium ions.

## Patentansprüche

1. Verfahren zur Herstellung einer $\alpha$, $\beta$ – ungesättigten Carbonsäure der allgemeinen Formel

$R^1(R^2)C{=}C(R^3)CO_2H$

in der jeder der Reste $R^1$, $R^2$ und $R^3$ unabhängig Wasserstoff, Fluor, Alkyl oder Fluoralkyl und mindestens einer der Reste $R^1$, $R^2$ und $R^3$ Fluor oder Fluoralkyl ist, dadurch gekennzeichnet, daß man
(a) ein Fluor enthaltendes Alkenylhalogenid der allgemeinen Formel

$R^1(R^2)C{=}C(R^3)X$

in der $R^1$, $R^2$ und $R^3$ die vorstehend angegebene Bedeutung haben und X Chlor, Brom oder Jod ist, mit Kohlendioxid in einem organischen Lösungsmittel und in Gegenwart von Zink und mindestens einem Kation aus der Gruppe der Erdalkalimetallionen, Alkalimetallionen und Ammoniumionen umsetzt und.
(b) das erhaltene Produkt hydrolysiert.

2. Verfahren nach Anspruch 1, wobei die Fluoralkylgruppe eine Perfluor- oder Polyfluor-aliphatische

Gruppe mit einer $C_{1-20}$ - unverzweigten oder verzweigten Kette ist.

3. Verfahren nach Anspruch 1 oder 2, wobei 1 bis 10 Äquivalente Zink pro Fluor enthaltendes Alkenylhalogenid verwendet werden.

4. Verfahren nach Anspruch 1, 2 oder 3, wobei die Umsetzung bei einer Temperatur von 0 bis 150°C durchgeführt wird.

5. Verfahren nach einem der Ansprüche 1 bis 4, wobei das Kation in einer Menge von 0,01 bis 50 Gramm Atome pro 1 Mol Fluor enthaltendes Alkenylhalogenid vorliegt.

6. Verfahren nach einem der Ansprüche 1 bis 5, wobei das Zink aktiviertes Zink ist.

7. Verfahren nach einem der Ansprüche 1 bis 6, wobei die Alkyl- oder Fluoralkylgruppe des Fluor enthaltenden Alkenylhalogenids 20 oder weniger Kohlenstoffatome enthält.

8. Verfahren nach einem der Ansprüche 1 bis 7, wobei das Fluor enthaltende Alkenylhalogenid 3,3,3-Trifluor-2-brompropen, 3,3,4,4,5,5,6,6,6-Nonafluor-2-bromhexen, 1,2,2-Trifluor-1-jodäthen, 1,2-Difluor-1-jod-3-methylpenten oder 2,3,3,4,4,5,5,6,6,7,7,8,8,9,9,9-hexadecafluor-1-jodnonen ist.

9. Verfahren nach einem der Ansprüche 1 bis 8, wobei das organische Lösungsmittel ausgewählt ist aus DMF, DMSO, N-Methylpyrrolidon, N,N-Dimethylacetamid, Tetramethylharnstoff und Hexamethylphosphorsäuretriamid.

10. Verfahren zur Herstellung eines $\alpha$, $\beta$ – ungesättigten Carbonsäuresalzes der allgemeinen Formel

$R^1(R^2)C{=}C(R^3)CO_2M$

in der jeder der Reste $R^1$, $R^2$ und $R^3$ unabhängig Wasserstoff, Fluor, Alkyl oder Fluoralkyl und mindestens einer der Reste $R^1$, $R^2$ und $R^3$ Fluor oder Fluoralkyl ist und M ein Kation darstellt, dadurch gekennzeichnet, daß man ein Fluor enthaltendes Alkenylhalogenid der allgemeinen Formel

$R^1(R^2)C{=}C(R^3)X$

in der $R^1$, $R^2$ und $R^3$ die vorstehend angegebene Bedeutung haben und X Chlor, Brom oder Jod ist, mit Kohlendioxid in einem organischen Lösungsmittel und in Gegenwart von Zink und mindestens einem Kation aus der Gruppe der Erdalkalimetallionen, Alkalimetallionen und Ammoniumionen umsetzt.

## Revendications

1. Procédé pour la préparation d'un acide carboxylique alpha-béta-insaturé répondant à la formule générale:

$R^1(R^2)C{=}C(R^3)CO_2H$

dans laquelle $R^1$, $R^2$ et $R^3$ représentent chacun de façon indépendante un atome d'hydrogène, un atome de fluor, un groupe alkyle, ou un groupe fluoroalkyle et au moins l'un de $R^1$, $R^2$ et $R^3$ est un atome de fluor ou un groupe fluoroalkyle, le procédé comprenant les étapes consistant à:

(a) faire réagir un halogénure d'alcényle contenant du fluor répondant à la formule générale suivante:

$R^1(R^2)C{=}C(R^3)X$

dans laquelle $R^1$, $R^2$ et $R^3$ sont les mêmes que ci-dessus et X est un atome de chlore, de brome ou d'iode, avec du bioxyde de carbone dans un solvant organique et en présence de zinc et au moins un cation choisi parmi les ions de métaux alcalino-terreux, les ions de métaux d'alcali et les ions d'ammonium et

(b) hydrolyser le produit obtenu.

2. Procédé selon la revendication 1, dans lequel le groupe fluoroalkyle est un groupe aliphatique perfluoro ou polyfluoro avec une chaîne rectiligne en $C_{1-20}$ ou ramifiée.

3. Procédé selon la revendication 1 ou 2, dans lequel on utilise entre 1 à 10 équivalents du zinc par rapport à l'halogénure d'alcényle contenant du fluor.

4. Procédé selon la revendication 1, 2 ou 3, dans lequel la réaction est exécutée à une température comprise entre 0 et 150°C.

5. Procédé selon l'une quelconque des revendications 1 à 4, dans lequel le cation est présent suivant une quantité comprise entre 0,01 et 50 atomes grammes par mole de l'halogénure d'alcényle contenant du fluor.

6. Procédé selon l'une quelconque des revendications 1 à 5, dans lequel le zinc est du zinc activé.

7. Procédé selon l'une quelconque des revendications 1 à 6, dans lequel le groupe alkyle ou fluoroalkyle de l'halogénure d'alcényle contenant du fluor comporte 20 atomes ou moins de carbone.

8. Procédé selon l'une quelconque des revendications 1 à 7, dans lequel l'halogénure d'alcényle contenant du fluor est le 3,3,3-trifluoro-2-bromopropène, le 3,3,4,4,5,5,6,6,6-nonafluoro-2-bromohexène, le 1,2,2,-trifluoro-1-iodiéthène, le 1,2-difluoro-1-iodo-3-méthylpentène, ou le 2,3,3,4,4,5,5,6,6, 7,7,8,8, 9,9,9-hexadécafluoro-1-iodononène.

9. Procédé selon l'une quelconque des revendications 1 à 8, dans lequel le solvant organique est choisi parmi le DMF, le DMSO, la N-méthylpyrrolidone, le N,N-diméthylacétamide, la tétraméthylurée, et l'hexaméthylphosphoramide.

10. Procédé pour la préparation d'un sel de carboxylate alpha-béta-insaturé répondant à la formule générale:

$R^1(R^2)C=C(R^3)CO_2M$

dans laquelle $R^1$, $R^2$ et $R^3$ représentent chacun de manière indépendante un atome d'hydrogène, un atome de fluor, un groupe alkyle ou un groupe fluoroalkyle et au moins l'un de $R^1$, $R^2$ et $R^3$ est un atome de fluor ou un groupe fluoroalkyle et M représente un cation, le procédé comprenant l'étape consistant à faire réagir un halogénure d'alcényle contenant du fluor répondant à la formule générale:

$R^1(R^2)C=C(R^3)X$

dans laquelle $R^1$, $R^2$ et $R^3$ sont les mêmes que ci-dessus et X est un atome de chlore, de brome ou d'iode, avec du bioxyde de carbone dans un solvant organique et en présence de zinc et au moins un cation choisi parmi les ions de métaux alcalino-terreux, les ions de métaux d'alcali et les ions ammonium.